# EUROPÄISCHE PATENTSCHRIFT

(11) **EP 3 573 594 B1**
(45) Veröffentlichungstag und Bekanntmachung des Hinweises auf die Patenterteilung: **19.08.2026**
(21) Anmeldenummer: 17816844.9
(22) Anmeldetag: 18.12.2017
(51) Int. Cl.: A61K 8/49, A61K 31/426, C07D 277/48, A61Q 19/00, A61K 8/34, A61K 9/00, A61K 9/06, A61K 9/107, A61K 31/045, A61K 47/34, C07D 277/46

(54) **WIRKSTOFFKOMBINATIONEN AUS TRANS-4-T-BUTYLCYCLOHEXANOL UND EINEM ODER MEHREREN ALKYLAMIDOTHIAZOLEN SOWIE KOSMETISCHE ODER DERMATOLOGISCHE ZUBEREITUNGEN, DIESE WIRKSTOFFKOMBINATIONEN ENTHALTEND**
ACTIVE INGREDIENT COMBINATIONS CONSISTING OF TRANS-4-T-BUTYLCYCLOHEXANOL AND ONE OR MORE ALKYLAMIDOTHIAZOLES AS WELL AS COSMETIC OR DERMATOLOGICAL PREPARATIONS CONTAINING SAID ACTIVE INGREDIENT COMBINATIONS
ASSOCIATIONS DE PRINCIPES ACTIFS CONSTITUÉES DE TRANS-4-T-BUTYLCYCLOHEXANOL ET D'UN OU DE PLUSIEURS AKYLAMIDOTHIAZOLS ET PRÉPARATIONS COSMÉTIQUES OU DERMATOLOGIQUES CONTENANT CES ASSOCIATIONS DE PRINCIPES ACTIFS

(30) Priorität: 24.01.2017 DE 102017201050
(43) Veröffentlichungstag der Anmeldung: 04.12.2019
(73) Patentinhaber: Beiersdorf AG, 22529 Hamburg (DE)
(72) Erfinder: NEUFANG, Gitta, 20149 Hamburg (DE); GROENNIGER, Elke, 22303 Hamburg (DE); WORTHMANN, Anne-Christin, 25474 Hasloh (DE); KOLBE, Ludger, 21255 Dohren (DE)
(74) Vertreter: Beiersdorf AG
(86) Internationale Anmeldenummer: PCT/EP2017/083304
(87) Internationale Veröffentlichungsnummer: WO 2018/137854

(56) Entgegenhaltungen:
- WO-A1-2014/139741
- WO-A2-2009/087242
- US-A1- 2016 324 832
- SYMRISE GMBH & CO KG ET AL: "Trans-tert-butyl cyclohexanol as skin irritation-reducing agent", RESEARCH DISCLOSURE, KENNETH MASON PUBLICATIONS, HAMPSHIRE, UK, GB, vol. 542, no. 17, 1 June 2009 (2009-06-01), pages 595, XP007139063, ISSN: 0374-4353

## Beschreibung

Die vorliegende Erfindung betrifft Wirkstoffkombinationen aus Trans-4-t-butylcyclohexanol und aus einem oder mehreren Alkylamidothiazolen sowie kosmetische oder dermatologische Zubereitungen, diese Wirkstoffkombinationen enthaltend.

Ziel der vorliegenden Erfindung war es somit, Wege zu finden, die Nachteile des Standes der Technik zu vermeiden. Insbesondere sollte die Wirkung der Hautpflegeprodukte physiologisch, schnell und nachhaltig sein.

Zwar kennt der Fachmann das Dokument "Trans-tert-butyl cyclohexanol as skin irritation-reducing aqent" (Symrise GmbH & Co KG et. al) Research Disclosure, Kenneth Mason Publications, Hampshire, UK, GB, Bd.542, Nr. 17, 1. Juni 2009, Seite 595, sowie die Schriften WO 2014/139741 A1, WO 2009/087242 A2 und US 2016/324832 A1 doch konnten diese nicht den Weg zur vorliegenden Erfindung weisen.

Unter Hautpflege im Sinne der vorliegenden Erfindung ist in erster Linie zu verstehen, dass ein angenehmes Hautgefühl herbeigeführt wird bzw. ein unangenehmes Hautgefühl vermindert wird.

Ein wichtiger Mediator für ein unangenehmes Hautgefühl ist der Neuromodulator wie CGRP (Calcitonin Gene-Related Peptide). Überraschenderweise zeigen Tyrosinaseinhibitoren, gewählt aus der Gruppe der Alkylamidothiazole und der TRPV1-Antagonist Trans-4-t-butylcyclohexanol eine synergistische Wirkung auf die Reduktion der Ausschüttung von CGRP (nach Capsaicinstimulus) was ein beruhigendes, angenehmes Hautgefühl zur Folge hat.

Trans-4-t-butylcyclohexanol, wird unter anderem von der Gesellschaft Symrise unter der Handelsbezeichnung "Symsitive^{®}" verkauft. Es zeichnet sich durch folgende chemische Struktur aus:

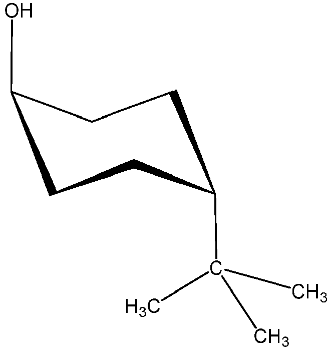

Es war überraschend und für den Fachmann nicht vorauszusehen, dass Wirkstoffkombinationen gemäß Anspruch 1 sowie kosmetische oder dermatologische Zubereitungen, diese Wirkstoffkombinationen enthaltend, den Nachteilen des Standes der Technik abhelfen. Wirkstoffkombinationen gemäß Anspruch 1 zeitigen eine synergistische Wirkung hinsichtlich der Ausschüttung von dem Neuromediator CGRP.

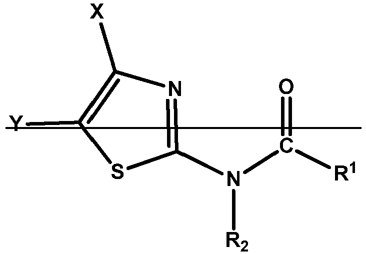

Die genannten Thiazole können sowohl als freie Base wie auch als Salz vorliegen: z.B. als Fluorid, Chlorid, Bromid, Iodid, Sulfat, Carbonat, Ascorbat, Acetat oder Phoshat. Im Besonderen als Halogensalze, wie z.B. Chlorid und Bromid.

Die Verbindungen 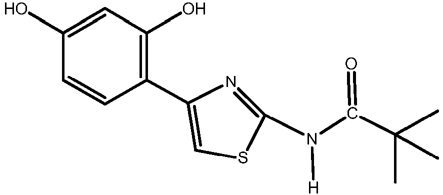
*N*-(4-(2,4-dihydroxyphenyl)thiazol-2-yl)pivalamide 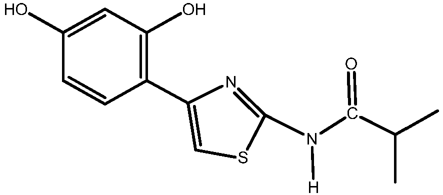
N-(4-(2,4-dihydroxyphenyl)thiazol-2-yl)isobutyramide 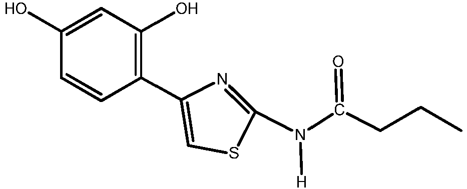
*N*-(4-(2,4-dihydroxyphenyl)thiazol-2-yl)butyramide 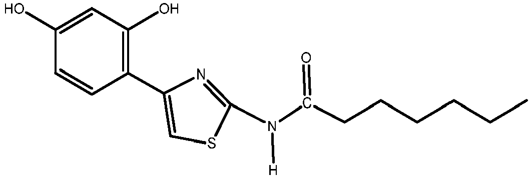
N-(4-(2,4-dihydroxyphenyl)thiazol-2-yl)heptanamide 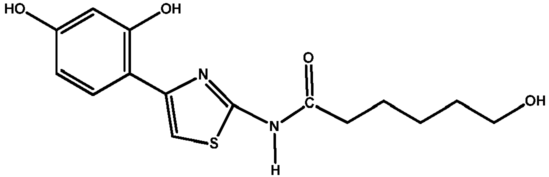
*N*-(4-(2,4-dihydroxyphenyl)thiazol-2-yl)-6-hydroxyhexanamide 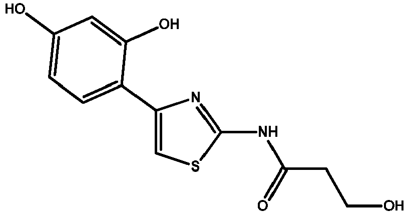
*N*-(4-(2,4-dihydroxyphenyl)thiazol-2-yl)-3-hydroxypropanamide 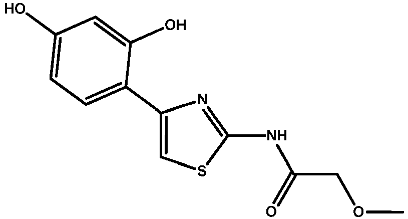
*N*-(4-(2,4-dihydroxyphenyl)thiazol-2-yl)-2-methoxyacetamide 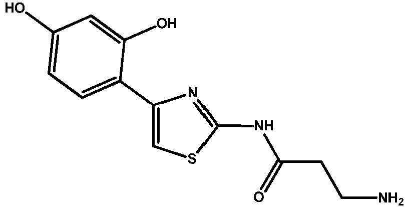
3-amino-N-(4-(2,4-dihydroxyphenyl)thiazol-2-yl)propanamide 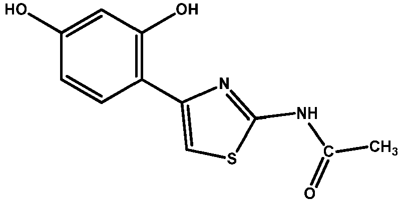
N-(4-(2,4-dihydroxyphenyl)thiazol-2-yl)acetamide 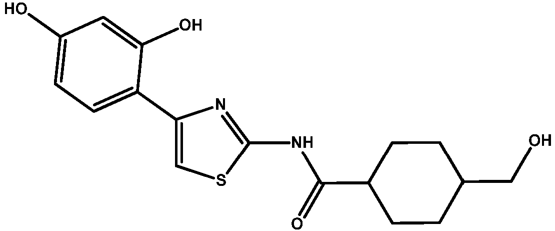
*N*-(4-(2,4-dihydroxyphenyl)thiazol-2-yl)-4-(hydroxymethyl)cyclohexanecarboxamide 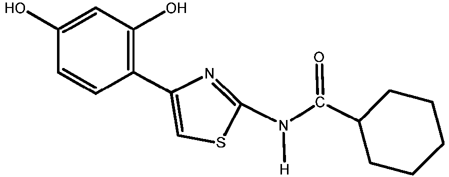
N-(4-(2,4-dihydroxyphenyl)thiazol-2-yl)cyclohexanecarboxamide 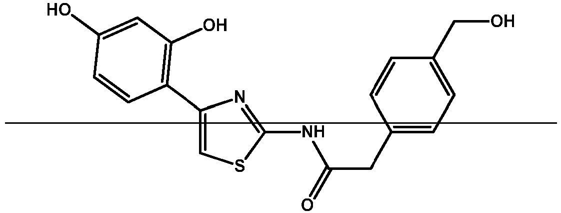
N-(4-(2,4-dihydroxyphenyl)thiazol-2-yl)-2-(4-(hydroxymethyl)phenyl)acetamide
sind erfindungsgemäß.

Erfindungsgemäße Zubereitungen, die erfindungsgemäßen Wirkstoffkombinationen enthaltend, sind besonders vorteilhaft dadurch gekennzeichnet, dass die erfindungsgemäßen Wirkstoffkombinationen in Konzentrationen von 0,05 bis 10,00 Gew.-%, bevorzugt 0,1 bis 5,0 Gew.-%, vorliegen, jeweils bezogen auf das Gesamtgewicht der Zubereitungen.

### Methodenbeschreibung der Wirksamkeitsuntersuchungen:

Die Wirksamkeit der Wirkstoffkombination wurde in vitro mit einem ELISA belegt. Hierzu wurden prokine Neuronen für 10 Minuten mit Capsaicin (50 nM) alleine und / oder in Kombination mit Trans-4-t-butylcyclohexanol und einem Alkylamidothiazolen stimuliert. Die Zellkulturüberstände der lediglich mit Capsaicin stimulierten Neuronen dienten als Kontrolle. Die Konzentration des ausgeschütteten Proteins CGRP wurde in den Zellkulturüberständen mittels "CGRP Enzyme ImmunoAssay Kit" von der Firma SPI-Bio, Montigny, Frankreich detektiert. Die Absorptionswerte wurde mit dem Gerät Spekta Max 250 von Molecular Devices, Ismaning, Deutschland gemessen.

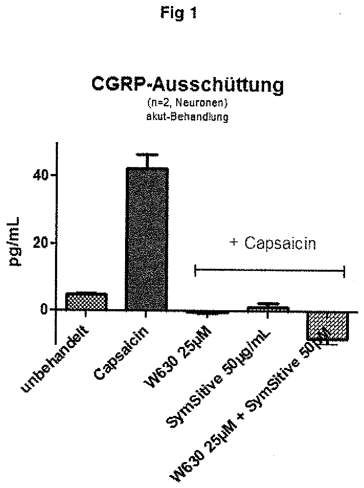

In Fig. 1 sind die Wirksamkeitsdaten für einige der beanspruchten Substanzen beispielhaft dargestellt. Daraus lässt sich schließen, dass die erfindungsgemäßen Kombination der Substanzen äußerst effektiv sind.

Vorteilhaft wären solche Zubereitungen, werden Gewichtsverhältnisse von der Gesamtmenge an aus einem oder mehreren Alkylamidothiazolen zu der Menge an Trans-4-t-butylcyclohexanol aus dem Bereich von 20 zu 1 bis 1 zu 20 gewählt, bevorzugt von 10 zu 1 bis 1 zu 10, insbesondere bevorzugt von 5 zu 1 bis 1 zu 5.

Bevorzugte Einsatzkonzentrationen von Trans-4-t-butylcyclohexanol in kosmetischen oder dermatologischen Zubereitungen werden aus dem Bereich von 0,000001 bis 10 Gew.-%, insbesondere 0,0001 bis 3 Gew.-%, ganz besonders 0,001 bis 1 Gew.-%, jeweils bezogen auf das Gesamtgewicht der Zubereitungen.

Vorteilhaft ist es, wenn solche Zubereitungen 0,000001 bis 10 Gew.-%, insbesondere 0,0001 bis 3 Gew.-%, ganz besonders 0,001 bis 1 Gew.-% an einem oder mehreren der erfindungsgemäß verwendeten Alkylamidothiazolen enthalten, bezogen auf das Gesamtgewicht der Zubereitung.

Erfindungsgemäße Zubereitungen können einen zusätzlichen Gehalt an Antioxidantien enthalten.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-,Cholesteryl- und Glycerylester sowie deren Salze, Dilaurylthiodipropionat, ferner (Metall)-Chelatoren (z.B. α-Hydroxyfettsäuren, Palmitinsäure, Phytinsäure), α-Hydroxysäuren (z.B. Citronensäure, Milchsäure, Apfelsäure), EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Rutinsäure und deren Derivate, α-Glycosylrutin, Ferulasäure, Furfurylidenglucitol, Butylhydroxytoluol, Harnsäure und deren Derivate, Harnstoff, Panthenol, Zink und dessen Derivate (z.B. ZnO),TiO₂, Resveratrol, Magnolienrindenextrakt (enthaltend Magnolol, Honokiol) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 10 Gew.-%, besonders bevorzugt 0,05 bis 5 Gew.-%, insbesondere 0,1 - 2 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 1 Gew.-%, bezogen auf das Gesamtgewicht der Formulierung, zu wählen.

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Es ist auch gegebenenfalls vorteilhaft, die Zusammensetzungen gemäß der Erfindung abzupuffern. Vorteilhaft ist ein pH-Bereich von 3,5 bis 8,0. Besonders günstig ist es, den pH-Wert in einem Bereich von 4,5 bis 7,5 zu wählen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nichtionische oder amphotere oberflächenaktive Substanz oder Gemische daraus, mindestens eine dialkylsubstituierte Carbonsäure im wässrigen Medium und Hilfsmittel, wie sie üblicherweise dafür verwendet werden.

Wie in der folgenden Versuchsanordnung herausgefunden wurde, reduzieren die erfindungsgemäßen Wirkstoffkombinationen bzw. Zubereitungen, solche Wirkstoffkombinationen enthaltend, die Ausschüttung von CGRP:
Die folgenden Beispiele sollen die Verkörperungen der vorliegenden Erfindungen verdeutlichen. Die Angaben beziehen sich stets auf Gew.-%, sofern nicht andere Angaben gemacht werden

| **INCI (Pflege)** | 1 | 2 | 3 | 4 | 5 |
|---|---|---|---|---|---|
| Natrium Cetearyl Sulfat | | 0,20 | | | |
| Glyceryl Stearate Citrate | | | | | 2,00 |
| Glyceryl Stearate | 3,00 | | | 2,00 | |
| PEG-40 Stearate | 1,00 | | | 2,00 | |
| Sodium Stearoyl Glutamate | | | 0,30 | | |
| Glyceryl Stearate SE | | 2,00 | | | |
| PEG-150 Distearate | 0,30 | | 0,80 | | |
| Cetearyl Alcohol | | 2,00 | | | 2,00 |
| Stearyl Alcohol | 1,00 | | 1,00 | 1,00 | 1,00 |
| Cetyl Alcohol | 1,00 | | | | |
| Hydriertes Kokosfettglyceride | | 1,00 | | | |
| Sheabutter | | | 1,00 | | |
| Xanthan Gum | 0,15 | | 0,20 | | |
| Acrylates/C10-30 Alkyl Acrylate Crosspolymer | 0,10 | 0,20 | | | |
| Cellulose | | | | 0,10 | |
| Ammonium Acryloyldimethyltaurate/ VP Copolymer | 0,30 | | | 0,50 | 0,50 |
| Dimethicone | 5,00 | | 2,00 | | |
| Isopropyl Stearate | | 3,00 | 3,00 | | |
| Sheabutter | 1,00 | 1,00 | | | 5,00 |
| C12-15 Alkylbenzoat | | | | 4,00 | |
| Sodium Hyaluronate | | | 1,00 | | |
| Polyquaternium-10 | | | | | 1,00 |
| Hydriertes Polydecen | | | | 1,00 | |
| Caprylsäure/Caprinsäure Triglycerid | | | 1,00 | | |
| Ethylhexylkokosfettsäureester | | 1,00 | | | |
| Füllstoffe / Additive (Stärke, Sio2, BHT, Talkum, Kaolin...) | 5,00 | | 7,00 | | 3,00 |
| Lichtfilter | 5,00 | | | | 8,00 |
| Ubichinon (Q10) | | 0,10 | | | |
| Isobutylamido Thiazolyl Resorcinol | | | | 0,30 | |
| Butylenglycol | | 2,00 | | | |
| Glycerin | 4,00 | 5,00 | 4,00 | 7,00 | 3,00 |
| Milchsäure | | 3,00 | | | |
| Carnitine | 0,50 | | | | |
| Glycyrrhiza Inflata Root Extract | | 0,03 | | | |
| Parfum | | | | 0,30 | |
| Trisodium EDTA | | 1,00 | | | 1,00 |
| Ethanol | 4,00 | | | | |
| Benzophenone-4 | | 0,10 | | | |
| Phenoxyethanol | 0,20 | | 0,60 | 0,40 | |
| N-(4-(2,4-Dihydroxyphenyl)thiazol-2-yl)-isobutyramid | 0,20 | 0.10 | 0.05 | 0.30 | 0,3 |
| N-(4-(2,4-Dihydroxyphenyl)thiazol-2-yl)-pivalamid | 0.01 | 0.25 | 0.15 | 0.10 | 0,1 |
| N-(4-(2,4-Dihydroxyphenyl)thiazol-2-yl)-butyramid | 0.25 | 0.15 | 0.30 | 0.35 | 0,35 |
| N-(4-(2,4-Dihydroxyphenyl)thiazol-2-yl)-cyclohexancarboxamid | 0.10 | 0.10 | 0.15 | 0.20 | 0,05 |
| Trans-4-t-butylcyclohexanol | 0,50 | 0,5 | 0, 2 | 0,5 | 0,3 |
| Aqua | ad 100 | | | | |

O/W -Emulsionen (aus dem Rohstoffpatent von W630 übernommen)

| **Rezepturbeispiel** | **1** | **2** | **3** | **4** |
|---|---|---|---|---|
| **Chemische/INCI-Bezeichnung** | **Gew.-%** | **Gew.-%** | **Gew.-%** | **Gew.-%** |
| Stearinsäure | 2,50 | 2,00 | 2,00 | 2,50 |
| Glyceryl Stearat | 1,00 | 1,00 | 1,00 | 1,00 |
| C12-15 Alkyl Benzoat | 3,00 | 5,00 | 3,00 | 2,00 |
| Caprylsäure/Caprinsäure Triglycerid | 2,50 | 2,50 | 2,00 | 2,50 |
| Isopropyl Palmitat | 2,00 | - | - | 2,00 |
| Cetylstearylalkohol | 3,00 | - | 2,00 | 3,00 |
| Cetyl Alkohol | - | 2,00 | - | - |
| Stearyl Alkohol | - | 2,00 | 1,00 | - |
| C13-16 Isoparaffin | - | - | - | 1,00 |
| Dibutyladioat | - | - | 1,50 | - |
| Cyclomethicon | 1,00 | 1,00 | 0,50 | - |
| Dicaprylyl Carbonat | 2,00 | 2,00 | 2,00 | 2,00 |
| Dimethicon | 1,00 | - | 0,50 | 1,00 |
| Glycerin | 5,00 | 7,00 | 5,00 | 9,00 |
| Ethylhexyl Cocoat | - | - | 1,00 | - |
| Methylparaben | 0,20 | - | - | - |
| Phenoxyethanol | 0,40 | 0,50 | 0,50 | 0,40 |
| Propylparaben | 0,10 | - | - | 0,10 |
| 1,2-Hexandiol | - | - | 0,10 | 0,10 |
| Ethylhexylglycerin | - | - | 0,20 | - |
| Methylisothiazolinon | - | 0,05 | - | - |
| Butylenglykol | - | - | 2,0 | - |
| Carbomer | 0,15 | 0,10 | 0,15 | 0,10 |
| Carrageenan | 0,10 | - | 0,10 | - |
| Xanthan Gummi | - | - | 0,10 | - |
| Acrylates/C10-30 Alkyl Acrylate Crosspolymer | - | 0,10 | - | 0,10 |
| Trinatrium EDTA | 0,20 | 0,20 | 0,20 | 0,20 |
| Tapioka Stärke | 1,50 | 1,00 | - | |
| Nylon-12 (1,8-Diazacyclotetradecan-2,7-dion Homopolymer) | - | 0,20 | - | 0,50 |
| Polymethylsilsesquioxane | - | 1,00 | 1,00 | - |
| Aluminum Stärke Octenylsuccinat | - | - | 1,00 | - |
| Distärkephosphat | 1,00 | 1,00 | - | 1,00 |
| Butyl Methoxydibenzoylmethan | 1,00 | 2,00 | 1,00 | 1,00 |
| Phenylbenzimidazole Sulfonsäure | 1,00 | 1,00 | 2,00 | 2,00 |
| Octocrylen | 2,00 | 2,00 | 1,00 | 2,00 |
| Ethylhexyl Salicylat | 1,00 | 1,00 | 2,00 | 1,00 |
| Hydroxypropyl tetrahydropyrantriol | 1,00 | 0,50 | - | - |
| Liponsäure | - | 0,50 | 0,20 | - |
| Kalium-Methoxysalicylate | 0,30 | - | 0,10 | 0,05 |
| Vitamin B6 HCl | 0,10 | 0,05 | - | 0,30 |
| Tranexamsäure | - | 0,01 | 0,25 | - |
| Pyrus Malus Stem Extract | 1,00 | 0,25 | 0,50 | 0,75 |
| N-(4-(2,4-Dihydroxyphenyl)thiazol-2-yl)-isobutyramid | 0,20 | 0.10 | 0.05 | 0.30 |
| N-(4-(2,4-Dihydroxyphenyl)thiazol-2-yl)-pivalamid | 0.01 | 0.25 | 0.15 | 0.10 |
| N-(4-(2,4-Dihydroxyphenyl)thiazol-2-yl)-butyramid | 0.25 | 0.15 | 0.30 | 0.35 |
| N-(4-(2,4-Dihydroxyphenyl)thiazol-2-yl)-cyclohexan-carboxamid | 0.10 | 0.10 | 0.15 | 0.20 |
| Natriumhydroxid | q.s. | q.s. | q.s. | q.s. |
| Trans-4-t-butylcyclohexanol | 0,50 | 0,5 | 0, 2 | 0,5 |
| Parfüm | 0,30 | 0,20 | 0,20 | 0,20 |
| Natriumhydroxid | q.s. | q.s. | q.s. | q.s. |
| Wasser | ad 100 | ad 100 | ad 100 | ad 100 |

Kommentar: Es handelt sich um fünf W/O Cremes mit verschiedenen Emulgatorsystemen

| **INCI (Pflege)** | **6** | **7** | **8** | **9** | **10** | **11** |
|---|---|---|---|---|---|---|
| Polyethylenglycol(21)stearylether | | | 2,00 | | | |
| Polyethylenglycol(2)stearylether | | | 1,00 | | | |
| Sorbitan Stearate | | 1,00 | | | | |
| Polyglyceryl-3-Methylglucosedistearat | | 3,00 | | | | |
| PEG-40 Stearate | 1,00 | | | | | |
| Glyceryl Stearate SE | 3,00 | | | | | |
| PEG-100 Stearate | | | | | 2,00 | |
| PEG-150 Distearate | | | | | 2,00 | |
| Cetearylglucosid | | | | 3,00 | | |
| Stearinsäure | | | | | | 2,50 |
| Cetearyl Alcohol | 2,00 | | | | | 2,00 |
| Stearyl Alcohol | | | | | | 1,00 |
| Myristyl Myristat | | | 1,50 | | | |
| Cetyl Alcohol | | 2,50 | | | | |
| Hydriertes Kokosfettglyceride | | | 1,00 | | | |
| Sheabutter | | | | 1,00 | | |
| Polyacrylsäure (Carbomer) | | | | | 0,50 | 0,40 |
| Xanthan Gum | | 0,15 | | | | |
| Acrylates/C10-30 Alkyl Acrylate Crosspolymer | 0,10 | | 0,30 | | | |
| Cellulose | | | | 2,00 | | |
| Ammonium Acryloyldimethyltaurate/ VP Copolymer | | | | 0,20 | | |
| Isopropyl Stearate | | 2,00 | | | | |
| Octyldodecanol | 5,00 | | 1,00 | | | |
| Sheabutter | | 3,00 | | | | |
| C12-15 Alkylbenzoat | | | 5,00 | | | |
| PEG-40 Hydrogenated Castor Oil | | | | | | 0,50 |
| Hydriertes Polydecen | | | 2,00 | | | |
| Dicaprylylether | 1,00 | | | 2,00 | | |
| C12-15 Alkylbenzoat | | 1,00 | | 1,00 | | |
| Füllstoffe / Additive (Stärke, Sio2, BHT, Talkum, Kaolin...) | | | | 10,0 | | |
| Pigmente (Titandioxid, Eisenoxid) | 5,00 | | 10,0 | | | |
| Lichtfilter | | | 30,0 | | 20,0 | |
| Butylenglycol | | | 4,00 | | | |
| Glycerin | 3,00 | 5,00 | 4,00 | 3,00 | 3,00 | 5,00 |
| Milchsäure | 2,00 | | | 5,00 | | |
| Carnitine | | 2,00 | | | | |
| Glycyrrhiza Inflata Root Extract | | | | | | 0,10 |
| Parfum | | | 0,20 | | | 0,10 |
| Trisodium EDTA | | | 0,50 | | | |
| Ethanol | | 3,00 | | | | |
| Phenoxyethanol | 0,60 | | 0,70 | | 0,80 | |
| N-(4-(2,4-Dihydroxyphenyl)thiazol-2-yl)-isobutyramid | 0,20 | 0.10 | 0.05 | 0.30 | 0.3 | 0.2 |
| N-(4-(2,4-Dihydroxyphenyl)thiazol-2-yl)-pivalamid | 0.01 | 0.25 | 0.15 | 0.10 | 0.1 | 0.01 |
| N-(4-(2,4-Dihydroxyphenyl)thiazol-2-yl)-butyramid | 0.25 | 0.15 | 0.30 | 0.35 | 0.35 | 0.25 |
| N-(4-(2,4-Dihydroxyphenyl)thiazol-2-yl)-cyclohexancarboxamid | 0.10 | 0.10 | 0.15 | 0.20 | 0,20 | 0,10 |
| Trans-4-t-butylcyclohexanol | 0,50 | 0,5 | 0, 2 | 0,5 | 0.5 | 0.2 |
| Aqua | ad 100 | | | | | |

## Patentansprüche

1. Wirkstoffkombinationen aus einem oder mehreren Alkylamidothiazolen und Trans-4-t-butylcyclohexanol, **dadurch gekennzeichnet, dass** das oder die Alkylamidothiazole folgende Struktur aufweisen: 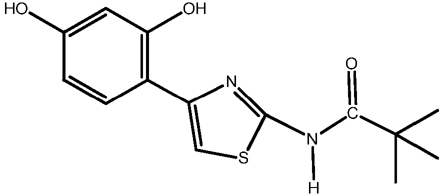
N-(4-(2,4-dihydroxyphenyl)thiazol-2-yl)pivalamide 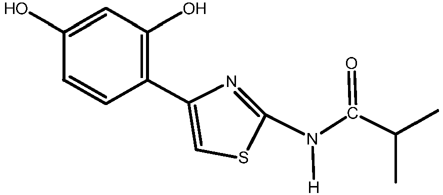
N-(4-(2,4-dihydroxyphenyl)thiazol-2-yl)isobutyramide 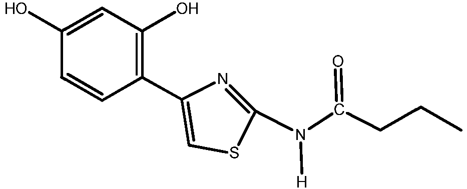
N-(4-(2,4-dihydroxyphenyl)thiazol-2-yl)butyramide 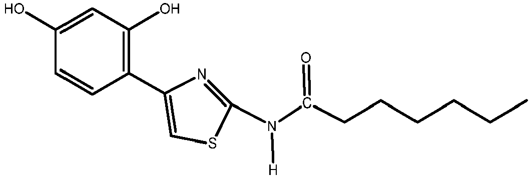
N-(4-(2,4-dihydroxyphenyl)thiazol-2-yl)heptanamide 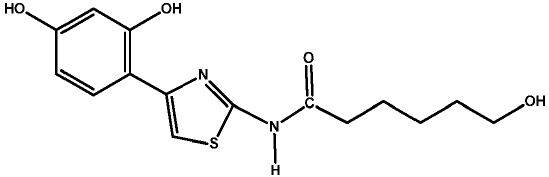
*N*-(4-(2,4-dihydroxyphenyl)thiazol-2-yl)-6-hydroxyhexanamide 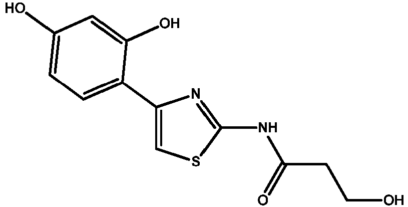
N-(4-(2,4-dihydroxyphenyl)thiazol-2-yl)-3-hydroxypropanamide 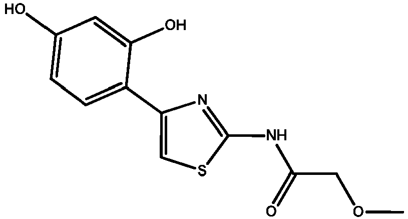
*N*-(4-(2,4-dihydroxyphenyl)thiazol-2-yl)-2-methoxyacetamide 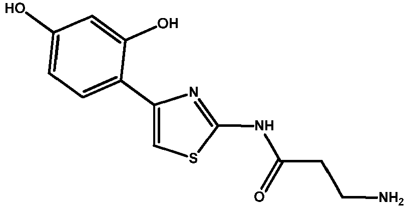
3-amino-N-(4-(2,4-dihydroxyphenyl)thiazol-2-yl)propanamide 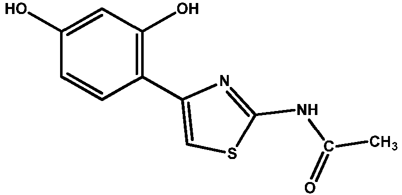
*N*-(4-(2,4-dihydroxyphenyl)thiazol-2-yl)acetamide 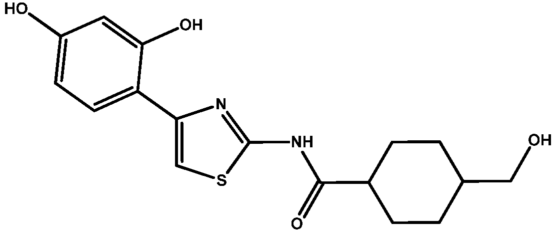
*N*-(4-(2,4-dihydroxyphenyl)thiazol-2-yl)-4-(hydroxymethyl)cyclohexanecarboxamide 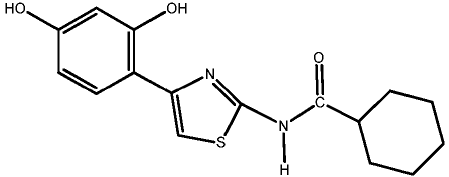
N-(4-(2,4-dihydroxyphenyl)thiazol-2-yl)cyclohexanecarboxamide
wobei das oder die Alkylamidothiazole sowohl als freie Base wie auch als kosmetisch und dermatologisch verwendbare Salze vorliegen können.

2. Wirkstoffkombinationen nach Anspruch 1, **dadurch gekennzeichnet, dass** der Gehalt an Alkylamidothiazolen in den kosmetischen oder dermatologischen Zubereitungen aus dem Bereich von 0,000001 bis 10,0 Gew.-%, insbesondere von 0,0001 bis 3,0 Gew.-% und ganz besonders von 0,001 bis 1 Gew.-% gewählt wird, jeweils bezogen auf das Gesamtgewicht der Zusammensetzung.

3. Wirkstoffkombinationen nach einem der vorstehenden Ansprüche, in denen Gewichtsverhältnisse der Gesamtmenge an einem oder mehreren Alkylamidothiazolen zur Menge an Trans-4-t-butylcyclohexanol aus dem Bereich von 20 zu 1 bis 1 zu 20 gewählt werden, bevorzugt von 10 zu 1 bis 1 zu 10, insbesondere bevorzugt von 5 zu 1 bis 1 zu 5.

4. Kosmetische oder dermatologische Zubereitungen, enthaltend Wirkstoffkombinationen nach einem der vorstehenden Ansprüche.

## Claims

1. Active ingredient combinations of one or more alkylamidothiazoles and trans-4-t-butyl-cyclohexanol, **characterized in that** the alkylamidothiazole(s) have the following structure: 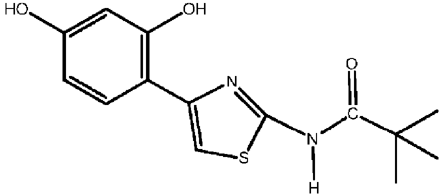
N-(4-(2,4-dihydroxyphenyl)thiazol-2-yl)pivalamide 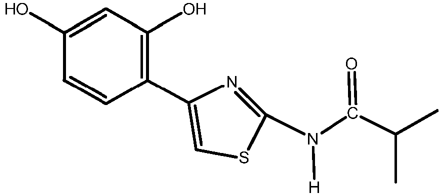
N-(4-(2,4-dihydroxyphenyl)thiazol-2-yl)isobutyramide 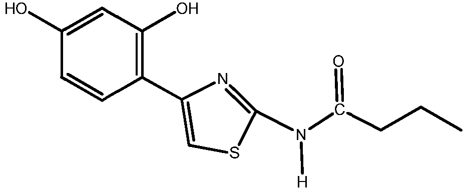
N-(4-(2,4-dihydroxyphenyl)thiazol-2-yl)butyramide 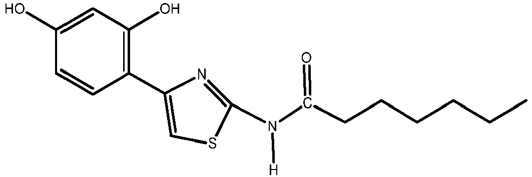
N-(4-(2,4-dihydroxyphenyl)thiazol-2-yl)heptanamide 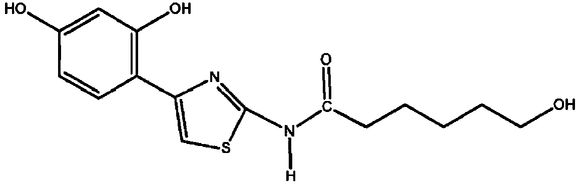
N-(4-(2,4-dihydroxyphenyl)thiazol-2-yl)-6-hydroxyhexanamide 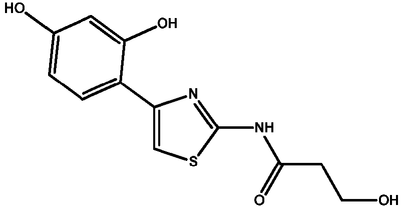
N-(4-(2,4-dihydroxyphenyl)thiazol-2-yl)-3-hydroxypropanamide 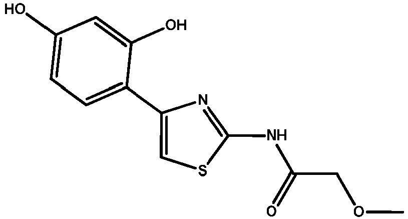
N-(4-(2,4-dihydroxyphenyl)thiazol-2-yl)-2-methoxyacetamide 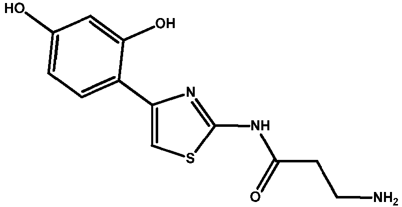
3-amino-N-(4-(2,4-dihydroxyphenyl)thiazol-2-yl)propanamide 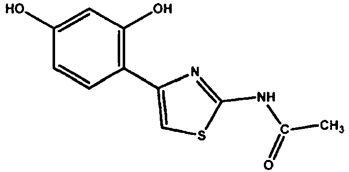
N-(4-(2,4-dihydroxyphenyl)thiazol-2-yl)acetamide 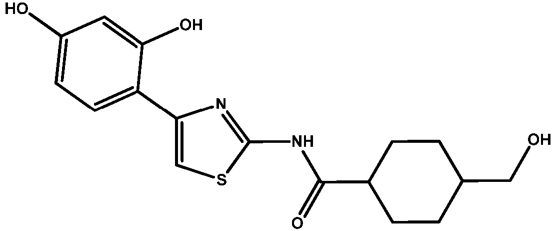
N-(4-(2,4-dihydroxyphenyl)thiazol-2-yl)-4-(hydroxymethyl)cyclohexanecarboxamide 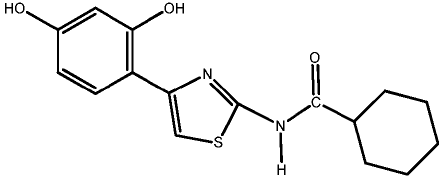
N-(4-(2,4-dihydroxyphenyl)thiazol-2-yl)cyclohexanecarboxamide
where the alkylamidothiazole(s) may be present either as the free base or as cosmetically and dermatologically usable salts.

2. Active ingredient combinations according to Claim 1, **characterized in that** the content of alkylamidothiazoles in the cosmetic or dermatological preparations is chosen from the range from 0.000001% to 10.0% by weight, in particular from 0.0001% to 3.0% by weight and very particularly from 0.001% to 1% by weight, based in each case on the total weight of the composition.

3. Active ingredient combinations according to any of the preceding claims, in which weight ratios of the total amount of one or more alkylamidothiazoles to the amount of trans-4-t-butylcyclohexanol are chosen from the range from 20:1 to 1:20, preferably from 10:1 to 1:10, especially preferably from 5:1 to 1:5.

4. Cosmetic or dermatological preparations comprising active ingredient combinations according to any of the preceding claims.

## Revendications

1. Combinaisons de principes actifs constituées par un ou plusieurs alkylamidothiazoles et du trans-4-t-butylcyclohexanol, **caractérisées en ce que** le ou les alkylamidothiazoles présentent la structure suivante : 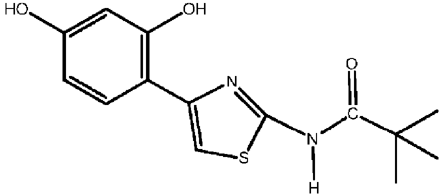
N-(4-(2,4-dihydroxyphényl)thiazol-2-yl)pivalamide 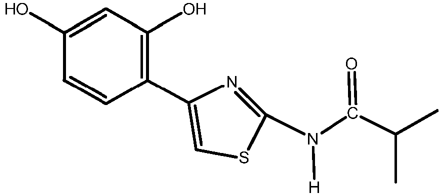
N-(4-(2,4-dihydroxyphényl)thiazol-2-yl)isobutyramide 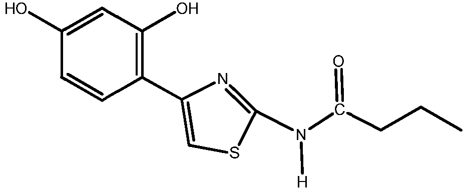
N-(4-(2,4-dihydroxyphényl)thiazol-2-yl)butyramide 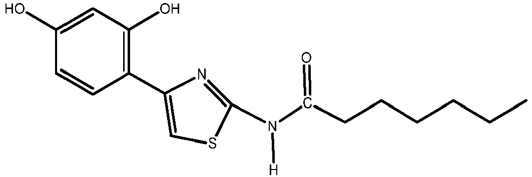
N-(4-(2,4-dihydroxyphényl)thiazol-2-yl)heptanamide 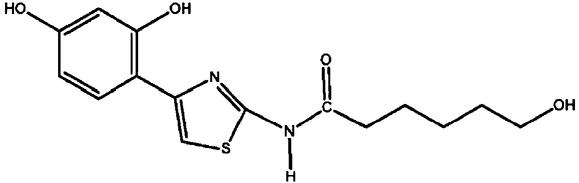
N-(4-(2,4-dihydroxyphényl)thiazol-2-yl)-6-hydroxyhexanamide 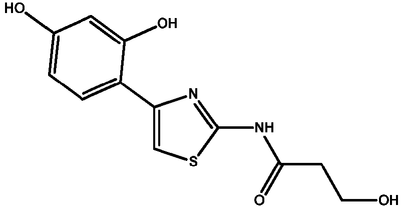
N-(4-(2,4-dihydroxyphényl)thiazol-2-yl)-3-hydroxypropanamide 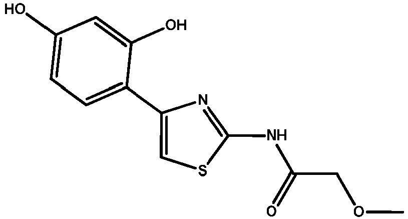
N-(4-(2,4-dihydroxyphényl)thiazol-2-yl)-2-méthoxyacétamide 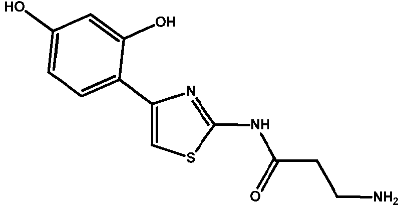
3-amino-N-(4-(2,4-dihydroxyphényl)thiazol-2-yl)propanamide 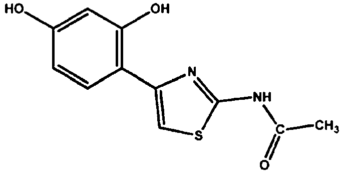
N-(4-(2,4-dihydroxyphényl)thiazol-2-yl)acétamide 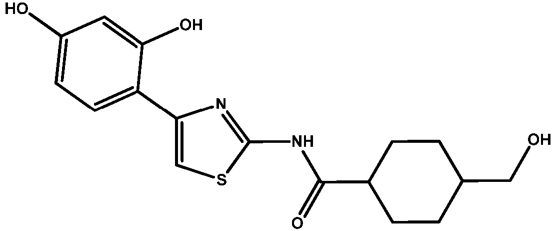
N-(4-(2,4-dihydroxyphényl)thiazol-2-yl)-4-(hydroxyméthyl)cyclohexanecarboxamide 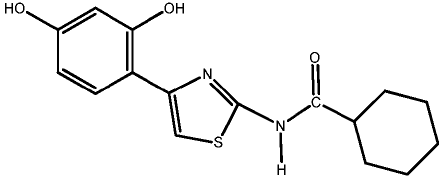
N-(4-(2,4-dihydroxyphényl)thiazol-2-yl)cyclohexanecarboxamide,
le ou les alkylamidothiazoles pouvant se trouver aussi bien sous forme de base libre que sous forme de sels utilisables en cosmétologie et en dermatologie.

2. Combinaisons de principes actifs selon la revendication 1, **caractérisées en ce que** la teneur en alkylamidothiazoles dans les préparations cosmétiques ou dermatologiques est choisie dans la plage de 0,000001 à 10,0% en poids, en particulier de 0,0001 à 3,0% en poids et tout particulièrement de 0,001 à 1% en poids, à chaque fois par rapport au poids total de la composition.

3. Combinaisons de principes actifs selon l'une des revendications précédentes, dans lesquelles les rapports en poids de la quantité totale d'un ou de plusieurs alkylamidothiazoles à la quantité de trans-4-t-butylcyclohexanol sont choisis dans la plage de 20:1 à 1:20, de préférence de 10:1 à 1:10, en particulier de préférence de 5:1 à 1:5.

4. Préparations cosmétiques ou dermatologiques contenant des combinaisons de principes actifs selon l'une des revendications précédentes.
